# EUROPEAN PATENT APPLICATION

(11) **EP 1 421 949 A1**
(43) Date of publication of application: **26.05.2004**
(21) Application number: 02760747.2
(22) Date of filing: 26.08.2002
(51) Int. Cl.: A61K 38/21, A61K 31/513, A61P 31/12, A61P 1/16

(54) **HEPATITIS B VIRUS PROLIFERATION INHIBITOR**

(30) Priority: 28.08.2001 JP 2001258030
(71) Applicant: TORAY INDUSTRIES, INC., Tokyo 103-8666 (JP)
(72) Inventor: ICHIDA, Takafumi, Niigata-shi, Niigata 950-0088 (JP); KAWASHIMA, Yoshiharu, Yokohama-shi, Kanagawa 227-0052 (JP)
(74) Representative: Kador & Partner
(86) International application number: PCT/JP2002/008576
(87) International publication number: WO 2003/020302

(57) **Abstract**

The present invention relates to an agent for suppression of lamivudine-resistant hepatitis B virus replication in long-term administration of lamivudine. The replication of hepatitis B virus is suppressed by administering IFN-β for a YMDD mutant virus appearing in long-term administration of lamivudine. Therefore, the agent is useful as a curative suppressor.

## Description

### Technical Field

The present invention relates to a medicine for suppression of lamivudine-resistant hepatitis B virus replication during long-term administration of lamivudine, the medicine comprising interferon-β (IFN-β) as an active ingredient.

### Background Art

Long-term administration of lamivudine for chronic hepatitis B causes the recurrence or breakthrough of hepatitis B with a probability of 50% or more due to a resistant virus produced by mutation in the YMDD motif of a hepatitis B virus polymerase gene. Combined therapy with interferon-α, strong neo-minofagen C (SNMC), or the like is currently conducted (Michio Sada, et al.: Lamivudine Therapy for Chronic Hepatitis B, Liver, Bile Duct and Pancreas, 41(1): 101-107, 2000), Hisaichi Tanigawa: Experience of Long-Term Dose of GG714 (Lamivudine) for Chronic Hepatitis B, Liver, Bile Duct and Pancreas, 41(1): 117-123, 2000).

However, it has been recognized that such a combination has an insufficient effect, and the replication of lamivudine-resistant hepatitis B virus takes place. Namely, it has become clear that there is a case in which hepatitis recurs due to the emergence of hepatitis B virus mutants during long-term administration of lamivudine, leading to a hepatic failure, and the emergence of a resistant virus becomes a problem. There is now no cure for sufficiently suppression of a resistant virus replication.

An object of the present invention is to provide a medicine for suppression of lamivudine-resistant hepatitis B virus replication during long-term administration of lamivudine.

### Disclosure of Invention

The present invention provides a curative medicine for suppression of lamivudine-resistant hepatitis B virus replication, the medicine comprising IFN-β as an active ingredient.

### Brief Description of the Drawings

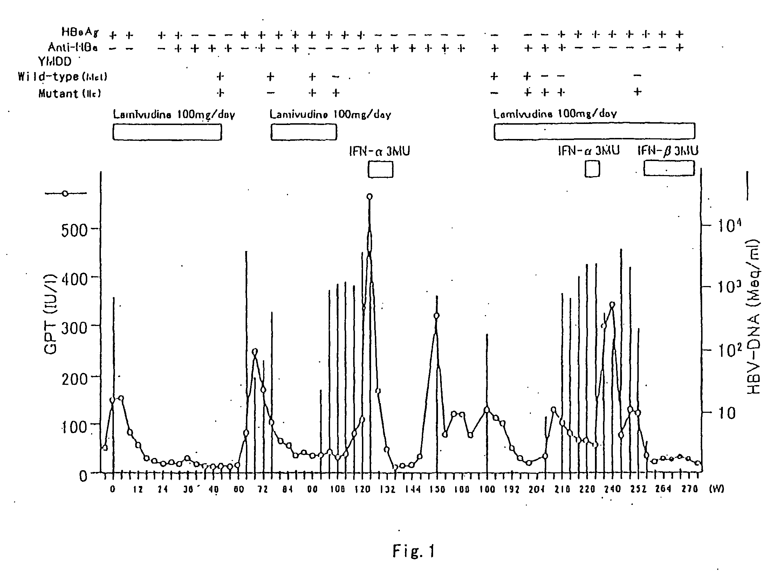

Fig. 1 shows the curative effect of IFN-β on a chronic hepatitis B case causing YMDD mutation during long-term administration of lamivudine.

### Best Mode for Carrying Out the Invention

In the present invention, natural IFN-β may be used, or IFN-β may be produced by chemical synthesis or genetic manipulation technology. In the present invention, natural IFN-β produced from human diploid fibroblasts is preferably used.

Natural IFN-β is characterized by its production method not using a tumorigenic cell strain and virus. Usually, cells are adsorbed and cultured on a glass or plastic surface or the surfaces of fine particles of DEAE-dextran, polyacrylamide or polystyrene.

The cultured IFN-β producing cells are efficiently produced in the culture solution by priming (In IFN-β production, the cells are pre-treated with a small amount of IFN-β to increase the amount of IFN-β produced, as compared with a case without pre-treatment. This shows that IFN itself is concerned in a production control mechanism for enhancing IFN production. It has been reported that priming increases the amount of IFN-mRNA and the transcription rate of IFN-mRNA.) and super-induction (The cells are stimulated with the synthetic double stranded RNA of an inducting agent Poly 1:Poly C, and then treated with actinomycin D (AMD) and cycloheximide (CH) serving as metabolic inhibitors to enhance IFN production. First, CH inhibits protein translation from mRNA to accumulate IFN-mRNA in the cells, and then CH is removed to synthesize IFN proteins. At this time, AMD is added for conversely inhibiting repressor mRNA to control a feedback system for inhibiting IFN synthesis. Although it is assumed that the repressor is present, the presence of the control system has been confirmed.) The thus-obtained product solution generally contains a low concentration of IFN-β, and many other foreign materials derived from the cells or additives. As a method for concentrating and purifying IFN-β, a chromatography method using an insoluble carrier bonded with a blue pigment and a metal chelate group-bonded carrier is preferably used. Namely, a solution containing crude IFN-β is brought into contact with the insoluble carrier bonded with the blue pigment, and then the IFN-β is recovered as a solution from the eluate. Then, the IFN-β solution is brought into contact with the chelate group-bonded carrier comprising a chelated metal of zinc or the like, and the IFN-β is recovered from the eluate to obtain concentrated and purified IFN-β.

If required, a stabilizer can be added to IFN-β used in the present invention. Examples of the stabilizer include human serum albumin, polyol disclosed in Japanese Unexamined Patent Application Publication No. 58-92619, an organic acid buffer disclosed in Japanese Unexamined Patent Application Publication No. 58-92621, and the like. Furthermore, in formulation, a carrier in common use may be appropriately mixed with IFN-β according to the administration method. As the dosage form, various forms such as an injection, a capsule, a transnasal agent, a suppository, an oral medicine, an ointment, and the like can be used. Although the dosage is properly determined according to the dosage object, the dosage method, and the symptom, the dosage is preferably in the range of 3,000,000 to 6,000,000 units/day.

In the present invention, lamivudine is administered to a patient with chronic hepatitis B with a dosage of 100-mg tablet per day. When lamivudine is administered for a long time of about 8 months or more to cause recurrence or breakthrough of hepatitis B due to the emergence of a YMDD mutant hepatitis B virus, IFN-β is administered in combination with lamivudine.

### [EXAMPLE]

The present invention will be described in further detail below with reference to an example.

### EXAMPLE 1

Confirmation of the effect of IFN administration to a patient with chronic hepatitis B:

Lamivudine was administered (first; 100 mg for 52 weeks) to a 54-year-old male patient with chronic hepatitis B. However, an increase in the amount of hepatitis B virus in the blood and the worsening (increases in HBV-DNA and GPT shown in Fig. 1) of the liver function were observed, and thus lamivudine was further administered (second; 100 mg for 32 weeks). During the administration of lamivudine, a resistant virus appeared (YMDD mutant YIDD+ shown in Fig. 1), and the amount of hepatitis B virus in the blood also increased.

Thereafter, the amount of hepatitis B virus in the blood remained high, and the liver function acutely worsened (GPT 567) about 3 months after the discontinuation of the administration. Therefore, 3,000,000 units of IFN-α were administered for a total of 28 days.

However, after the termination of the administration, the amount of hepatitis B virus in the blood again increased, and the liver function worsened. Therefore, lamivudine was further administered (third; 100 mg for 98 weeks). 18 weeks after the start of the administration, a resistant virus (YMDD mutant YIDD) appeared, and an increase in the amount of hepatitis B virus in the blood and the worsening of the liver function were again observed. Although 3,000,000 units of IFN-α were administered in combination with lamivudine, the liver function further worsened, and thus the administration of IFN-α was discontinued.

Thereafter, hepatitis B virus in the blood remained positive, and the liver function remained abnormal. Therefore, 3,000,000 units of IFN-β were intravenously administered for 30 weeks. At the termination of the IFN-β administration, hepatitis B virus in the blood became negative, and the liver function was normalized (Fig. 1). About 4 months after the termination of administration, hepatitis B virus in the blood remained negative, and the liver function also remained normal.

### Industrial Applicability

The suppressing hepatitis B virus replication agent comprising interferon-β as an active ingredient of the present invention exhibits a suppressing effect on lamivudine-resistant hepatitis B virus replication during long-term administration of lamivudine, and is thus useful as a suppressing viral replication agent for lamivudine-resistant hepatitis B virus.

## Claims

1. An agent for suppression of hepatitis B virus replication when the recurrence or breakthrough of chronic hepatitis B occurs due to the emergence of a resistant virus during long-term administration of lamivudine, the agent comprising interferon-β as an active ingredient.

2. The agent for suppression of hepatitis B virus replication according to claim 1, wherein the interferon-β is of a natural type.

3. A method for suppression of hepatitis B virus replication comprising administering interferon-β to a patient when the recurrence or breakthrough of chronic hepatitis B occurs during long-term administration of lamivudine.

4. The method for suppression of hepatitis B virus replication according to claim 3, wherein the interferon-β is of a natural type.

5. The method for suppression of hepatitis B virus replication according to claim 3 or 4, wherein the dosage of the interferon-β is 3,000,000 to 6,000,000 units/day.

6. The use of interferon-β for producing an agent for suppression of hepatitis B virus replication when the recurrence or breakthrough of chronic hepatitis B occurs due to the emergence of a resistant virus during long-term administration of lamivudine.
